# EUROPEAN PATENT APPLICATION

(11) **EP 3 683 299 A1**
(43) Date of publication of application: **22.07.2020**
(21) Application number: 18856642.6
(22) Date of filing: 14.09.2018
(51) Int. Cl.: C12M 1/34, C08F 214/18, B81B 1/00

(54) **MICROCHANNEL CHIP**

(30) Priority: 15.09.2017 JP 2017177979
(71) Applicant: AGC Inc., Chiyoda-ku Tokyo 100-8405 (JP)
(72) Inventor: OHKURA Masahiro, Tokyo 100-8405 (JP); TAGUCHI Daisuke, Tokyo 100-8405 (JP); IDE Masamichi, Tokyo 100-8405 (JP); MATSUOKA Naoki, Tokyo 100-8405 (JP)
(74) Representative: Müller-Boré & Partner Patentanwälte PartG mbB
(86) International application number: PCT/JP2018/034244
(87) International publication number: WO 2019/054500

(57) **Abstract**

To provide a microchannel chip which is a chip provided internally with an inner space that can be used as a flow channel, and which is excellent in drug non-absorption properties of the channel. The microchannel chip has formed therein a plurality of openings H1, H2 and an inner space S connecting the plurality of openings H1, H2, and at least a part of the surface in contact with the inner space S is made of a fluorinated copolymer containing from 5 to 99 mol% of units based on CF₂=CFX (X is a fluorine atom, a chlorine atom or CF₃).

## Description

### TECHNICAL FIELD

The present invention relates to a microchannel chip having provided an inner space that can be used as a flow channel.

### BACKGROUND ART

In a test of carrying out toxicity evaluation of e.g. a chemical against animals, a device is required which stores cells in a living state so that a drug is supplied there to via a flow channel to carry out observation and evaluation by letting the cells be in contact with the drug while culturing the cells. Patent Document 1 discloses a method for culturing by supplying a drug and cells in a flow channel by using a microfluidic device having a flow channel therein at the time of carrying out functional evaluation of an animal experiment drug.

### PRIOR ART DOCUMENT

### PATENT DOCUMENT

Patent Document 1: WO2016/104541

### DISCLOSURE OF INVENTION

### TECHNICAL PROBLEM

In Patent Document 1, the material for the microfluidic device is not particularly considered, and in paragraph 0083, a common polymer such as polydimethylsiloxane is exemplified.

However, the material for such a device is required to have softness like an organ tissue, and transparency so that the state of cells can be observed from the outside, and at the time of evaluating a drug, if the material forming the flow channel in the device absorbs the drug or reacts with the drug, accurate evaluation cannot be done.

The present invention has an object to provide a microchannel chip (hereinafter simply referred to also as a chip) having provided an inner space that can be used as a flow channel, which is suitable as a microfluidic device to be used for e.g. toxicity evaluation of e.g. a drug against animals, and of which the material forming the flow channel in the device has softness and transparency, and is excellent in non-absorbability of a drug.

### SOLUTION TO PROBLEM

The present invention is one to achieve the above object and has the following embodiments.
[1] A chip having formed a plurality of openings and an inner space connecting the plurality of openings, wherein at least a part of the surface in contact with the inner space is made of a fluorinated copolymer containing from 5 to 99 mol% of units based on CF₂=CFX (X is a fluorine atom, a chlorine atom or CF₃), to all units in the copolymer.
[2] The chip according to [1], wherein the inner space includes a storage portion for storing cells.
[3] The chip according to [1] or [2], wherein at least a first substrate, a second substrate and a third substrate are laminated in this order, wherein in the first substrate, a plurality of through holes are formed, and in the second substrate, a through groove penetrating from the front surface to the back surface is formed so as to connect the plurality of through holes of the first substrate,
   at least one of the first substrate, the second substrate and the third substrate is made of the fluorinated copolymer, or
   at least a part of the inner wall surfaces of the plurality of through holes and the surface facing the through groove, of the first substrate, the inner wall surface of the through groove of the second substrate, and the surface facing the through groove, of the third substrate, is coated with the fluorinated copolymer.
[4] The chip according to [3], wherein the inner space formed by the plurality of through holes and the through groove, incudes a storage portion for storing cells.
[5] The chip according to [1] or [2], wherein at least a first substrate and a second substrate are laminated, wherein in the first substrate, a plurality of through holes are formed, and in the second substrate, a bottomed groove is formed so as to connect the plurality of through holes of the first substrate,
   at least one of the first substrate and the second substrate is made of the fluorinated copolymer, or
   at least a part of the inner wall surfaces of the plurality of through holes, and the surface facing the groove, of the first substrate, and the bottom surface and inner wall surface of the groove of the second substrate, is coated with the fluorinated copolymer.
[6] The chip according to [5], wherein the inner space formed by the plurality of through holes and the bottomed groove, incudes a storage portion for storing cells.
[7] The chip according to [1] or [2], wherein at least a first substrate and a second substrate are laminated, wherein in the first substrate, a plurality of through holes are formed and a bottomed groove is disposed so as to connect the through holes, and in the second substrate, no hole or groove is formed,
   at least a part of the inner wall surfaces of the plurality of through holes and the surface facing the groove, of the first substrate, is made of the fluorinated copolymer, or
   at least a part of the inner wall surfaces of the plurality of through holes and the surface facing the groove, of the first substrate, is coated with the fluorinated copolymer.
[8] The chip according to [1] or [2], wherein at least a first substrate and a second substrate are laminated, wherein in the first substrate, a plurality of through holes and a bottomed groove are formed, and in the second substrate, a bottomed groove is disposed so as to connect the plurality of through holes of the first substrate, and between the first and second substrate, a film is disposed so as not to block the through holes,
   at least a part of the inner wall surfaces of the plurality of through holes and the surface facing the bottomed groove, of the first substrate, and the bottom surface and inner wall surface of the groove of the second substrate, is made of the fluorinated copolymer, or
   at least a part of the inner wall surfaces of the plurality of through holes of the first substrate, and the bottom surface and inner wall surface of the groove of the second substrate, is coated with the fluorinated copolymer.
[9] The chip according to [8], wherein the film as defined in [8] is made of collagen, polydimethylsiloxane, or a fluorinated copolymer containing from 5 to 99 mol% of units based on CF₂=CFX (X is a fluorine atom, a chlorine atom or CF₃).
[10] The chip according to any one of [1] to [9], wherein the fluorinated copolymer is a copolymer having from 50 to 80 mol% of units based on tetrafluoroethylene, the transmission coefficient of oxygen is from 1.0 to 390×10⁻¹⁰ [cm^{3∗}cm/(cm^{2∗}s^{∗}cmHg)], and the transmission coefficient of carbon dioxide is from 5.0 to 1,900×10⁻¹⁰ [cm^{3∗}cm/(cm^{2∗}s^{∗}cmHg)].
[11] The chip according to any one of [1] to [10], wherein the fluorinated copolymer is a copolymer containing from 50 to 70 mol% of units based on tetrafluoroethylene, the type A durometer hardness is at least 80, the Young's modulus is at most 1.5 MPa, the nifedipine absorptivity is at most 30%, the BayK8644 absorptivity is at most 10%, and the inner space of the chip can be visually confirmed.
[12] The chip according to any one of [1] to [11], wherein the units based on said CF₂=CFX are units based on tetrafluoroethylene, units based on hexafluoropropylene, or units based on chlorotrifluoroethylene.
[13] The chip according to any one of [1] to [12], wherein the fluorinated copolymer further contains at least one type selected from the group consisting of units based on propylene, units based on ethylene, units based on vinylidene fluoride, and units based on a perfluoro(alkyl vinyl ether), in an amount of from 1 to 95 mol% to all units which the polymer comprises.
[14] The chip according to any one of [1] to [13], wherein the fluorinated copolymer is either
   a copolymer comprising units based on tetrafluoroethylene and units based on propylene, and their total is from 65 to 100 mol% to all units,
   a copolymer comprising units based on tetrafluoroethylene and units based on ethylene, and their total is from 80 to 100 mol% to all units,
   a copolymer comprising units based on hexafluoropropylene and units based on vinylidene fluoride, and their total is from 50 to 100 mol% to all units,
   a copolymer comprising units based on chlorotrifluoroethylene and units based on ethylene, and their total is from 80 to 100 mol% to all units,
   a copolymer comprising units based on tetrafluoroethylene and units based on a perfluoro(alkyl vinyl ether), and their total is from 50 to 100 mol%, or
   a copolymer comprising units based on tetrafluoroethylene and units based on hexafluoropropylene, and their total is from 50 to 100 mol% to all units.
[15] The chip according to any one of [1] to [14], wherein the fluorinated copolymer has from 0.01 to 5.0 mass% of iodine atoms, to the copolymer.
[16] The chip according to any one of [1] to [15], wherein the fluorinated copolymer comprises units based on tetrafluoroethylene and units based on propylene, and their total is from 65 to 100 mol% to all units,
   the molar ratio of units based on tetrafluoroethylene/units based on propylene is from 30/70 to 70/30, and the copolymer contains from 0.01 to 5.0 mass% of iodine atoms.
[17] The chip according to any one of [1] to [16], wherein the fluorinated copolymer comprises units based on tetrafluoroethylene and units based on a perfluoro(alkyl vinyl ether), and their total is from 50 to 100 mol% to all units,
   the molar ratio of units based on tetrafluoroethylene/units based on a perfluoro(alkyl vinyl ether) is from 5/95 to 95/5, and
   the copolymer contains from 0.1 to 20 mol% of units derived from a monomer III represented by the following formula (III),

   CR¹¹R¹²=CF-Q-R¹³-CO-Z (III)

   (in the formula (III), R¹¹ and R¹² are each independently a hydrogen atom or a fluorine atom, Q is a single a bond or an etheric oxygen atom, R¹³ is a group having an etheric oxygen atom at least at one terminal or between a carbon-carbon bond of a fluoroalkylene group or two or more fluoroalkylene groups, -Z is -OH, -OR¹⁴, -NR¹⁵R¹⁶, -NR¹⁷NR¹⁸H or NR¹⁹OR²⁰, R¹⁴ is an alkyl group, and R¹⁵, R¹⁶, R¹⁷, R¹⁸, R¹⁹ and R²⁰ are each independently a hydrogen atom or an alkyl group.)
[18] The chip according to any one of [1] to [17], which contains cells in the inner space.
[19] The chip according to [18], wherein the fluorinated copolymer is a copolymer containing from 50 to 70 mol% of units based on tetrafluoroethylene, and elongation of the cell culture surface in the state of containing cells in the inner space is from 1.0 to 25.0%.

The microchannel chip of the present invention has an inner space that can be used as a flow channel, in which the material forming the flow channel has softness and transparency, and is excellent in non-absorbability of a drug, and the flow channel is excellent in non-absorbability of a drug.

### BRIEF DESCRIPTION OF THE DRAWINGS

Fig. 1 is a schematic perspective view of a first embodiment of the chip of the present invention.
Fig. 2 is a schematic cross-sectional view of the chip in Fig. 1.
Fig. 3 is a schematic plan view of three substrates, as the chip in Fig. 1 has been disassembled.
Fig. 4 is a schematic cross-sectional view showing a third embodiment of the chip of the present invention.
Fig. 5 is a schematic plan view of two substrates, as the chip in Fig. 4 has been disassembled.
Fig. 6 is a schematic cross-sectional view showing a fifth embodiment of the chip of the present invention.
Fig. 7 is a schematic plan view, as the chip in Fig. 6 has been disassembled.
Fig. 8 is a schematic cross-sectional view showing a seventh embodiment of the chip of the present invention.
Fig. 9 is a schematic plan view, as the chip in Fig. 8 has been disassembled.
Fig. 10 shows specific example structures of seventh and eighth embodiments of the chip of the present invention.
Fig. 11 is a fluorescence micrograph showing the drug non-absorbability evaluation results in Experimental Example 1.
Fig. 12 is a fluorescence micrograph showing the drug non-absorbability evaluation results in Comparative Example 1.
Fig. 13 is a fluorescence micrograph showing the drug non-absorbability evaluation results in Experimental Example 2.
Fig. 14 is a fluorescence micrograph showing the drug non-absorbability evaluation results in Experimental Example 3.
Fig. 15 is a fluorescence micrograph showing the drug non-absorbability evaluation results in Experimental Example 4.
Fig. 16 is a fluorescence micrograph showing the drug non-absorbability evaluation results in Experimental Example 5.
Fig. 17 is a fluorescence micrograph showing the drug non-absorbability evaluation results in Experimental Example 6.
Fig.18 is a fluorescence micrograph showing the drug non-absorbability evaluation results in Experimental Example 7.

### DESCRIPTION OF EMBODIMENTS

The following definitions of terms apply throughout the specification and claims.

A "unit derived from a monomer" is the general term for an atomic group which is formed directly by polymerization of one molecule of a monomer and an atomic group obtainable by chemically converting a portion of the atomic group.

An "etheric oxygen atom" means an oxygen atom forming an etheric bond (-O-) between carbon-carbon atoms.

A "perfluoroalkylene group" means a group in which hydrogen atoms covalently bonded to carbon atoms of an alkylene group have been all substituted by fluorine atoms.

A numerical range represented by an expression " to " means a numerical range including the numerical values before and after the expression to be the lower limit value and the upper limit value.

### <First embodiment>

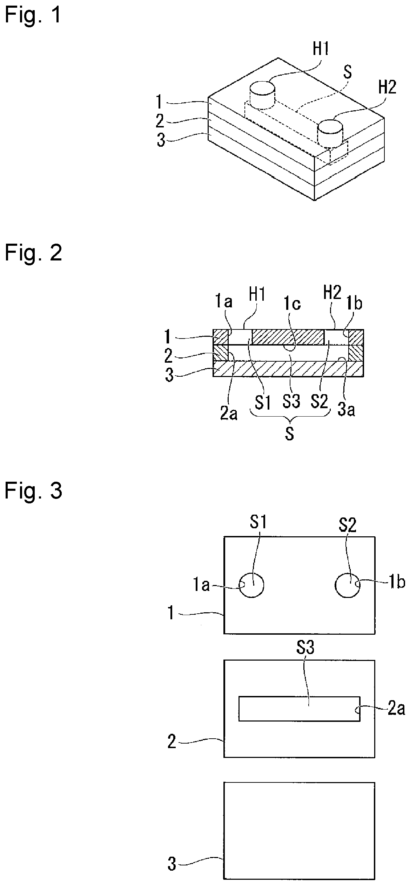

Fig. 1 is a schematic perspective view showing a first embodiment of the chip of the present invention, and Fig. 2 is a schematic cross-sectional view of the chip in Fig. 1. 1 to 3 show first to third substrates, respectively. Fig. 3 is a schematic plan view wherein the chip is disassembled into the first to third substrates. In this embodiment, the first to third substrates are made of a fluorinated copolymer.

In the chip of this embodiment, the first substrate 1, the second substrate 2 and the third substrate 3 are laminated in this order. In the first substrate 1, two through holes S1, S2 are formed. The open ends on the opposite side to the second substrate 2, of the through holes S1, S2, are openings H1, H2. 1a shows the inner wall surface of the through hole S1, and 1b shows the inner wall surface of the through hole S2.

The second substrate 2 is provided with a through groove S3 penetrating from the front surface to the back surface. The through groove S3 is provided so that when the first substrate 1 and the second substrate 2 are laminated, the through groove S3 connects the through holes S1, S2. The front and back surfaces of the third substrate 3 are flat surfaces. Reference numeral 1c denotes a surface facing the through groove S3, of the first substrate 1, and 3a denotes a surface facing the through groove S3, of the third substrate 3.

In the chip having the first to third substrates 1 to 3 laminated, the space formed by the through holes S1, S2 and the through groove S3, is the inner space S for connecting the openings H1, H2.

The chip of this embodiment can be used as a vessel for culturing cells in the inner space S. In that case, the inner space S includes a storage portion (not shown) for storing cells. For example, such a storage portion may be provided on the surface of the surface 3a facing the through groove S3, of the third substrate 3.

The size of the chip is not limited, but, when, in the surface of the first substrate 1, the direction through the centers of two openings H1, H2 is taken as the length, the direction orthogonal to the length direction is taken as the width, and the direction orthogonal to the length direction and width direction, is taken as the thickness, for example, the length of the chip is from 10 to 100 mm, the width is from 10 to 100 mm, and the thickness is from 100 to 20,000 µm. The total volume of the inner space S for connecting the two openings H1, H2, is, for example, from 10 to 2,000 µL.

The inner diameter of the openings H1, H2 is, for example, from 100 to 3,000 µm.

The respective thicknesses of the first to third substrates 1 to 3 may be the same or may be different from one another. The thickness of each substrate may, for example, be preferably from 200 to 10,000 µm.

The first to third substrates are preferably transparent, since it is thereby easy to observe the inside. For example, the transmittance of each substrate is preferably at least 80% at a wavelength of 400 nm.

Similarly, the haze value measured by a haze meter in accordance with JIS-K-7136 is preferably at most 50%, more preferably at most 30%, further preferably at most 20%.

Among the first to third substrates, at least one preferably has flexibility. When the chip has flexibility, it is possible to impart a mechanical force to cells in the inner space by deforming the chip as the case requires. For example, to cells in culture, it is possible to apply mechanical stretch that mimics the motion of an organ. For example, the Type A durometer hardness of the substrate is preferably at most 80, more preferably at most 60.

The chip of this embodiment can be produced, for example, by the following method.

Three films made of a fluorinated copolymer are prepared and used as the first to third substrates. In the first substrate 1, two through holes S1, S2 penetrating from the front surface to the back surface will be provided. In the second substrate 2, a through groove S3 penetrating from the front surface to the back surface will be provided. The third substrate 3 will not be processed.

So that the through groove S3 will connect the two through holes S1, S2, the first substrate 1, the second substrate 2 and the third substrate 3 will be laminated in this order from the top, and the substrates will be bonded to one another in a liquid-tight manner to obtain the chip.

As the method for bonding the substrates to one another, a method of heating to heat-seal the substrates to one another, a method of superimposing the substrates to one another, and further crosslinking them by ultraviolet irradiation, radiation irradiation, electron beam irradiation or heating, a method of providing an adhesive layer between the substrates, a method of activating the bonding surface by corona discharge irradiation, plasma irradiation, ultraviolet irradiation, ozone exposure, base exposure, etc., followed by lamination, or a method in which two or more of these methods are combined, may be exemplified.

The chip of this embodiment is preliminarily subjected to sterilization treatment when cells are to be put therein. As a method of sterilizing the chip, a method of applying treatment such as a method of applying ultraviolet irradiation, radiation irradiation, electron beam irradiation, corona discharge irradiation, plasma irradiation, ozone exposure, base exposure, steam-pressured sterilization (autoclave), etc. to the chip, or a method of putting the chip into an environment containing a sterilizing gas such as ethylene oxide, may be exemplified.

The surface in contact with the inner space, of the chip, may be used as it is, but surface modification may be applied so that adhesion of cells may be controlled. For example, at least the surface to be the storage portion of cells may be surface-modified to improve the adhesion of cells. Further, the surface not to store cells may be surface modified so as to prevent the adhesion of cells or a drug.

As a method for surface modification, with respect to the surface in contact with the inner space, a method of applying treatment such as ultraviolet irradiation, radiation irradiation, electron beam irradiation, corona discharge irradiation, plasma irradiation, ozone exposure, base exposure, etc., or a method of coating the flow channel surface with a hydrophilic or water-repellent compound, may be exemplified. As the hydrophilic or water-repellent compound, a phospholipid polymer, polyhydroxyethyl methacrylate, polyacrylamide, polyvinyl acetate, polyvinyl alcohol, polyethylene glycol, polyurea, polyurethane, agarose, chitosan, albumin, polydimethylsiloxane, or perfluoropolyether may be exemplified. In such a compound, from such a viewpoint that it is possible to improve adhesion to the flow channel surface, it is preferred that a portion of the chemical structure, particularly a main chain terminal, is functionalized to be a silane coupling group, etc. For example, Dow Corning product name, 2634 Coating, may be mentioned.

Further, coating treatment with a protein or a peptide may also be mentioned. Examples of the protein or peptide may be collagen, gelatin, fibronectin, vitronectin, laminin, albumin, RGD peptide, etc., but are not limited thereto. Also, a combination of two or more of these methods may be mentioned.

Types of cells are not limited. The morphology of cells may be single cells, or may be cell aggregates having a plurality of cells gathered. Or, it may be a biological tissue which has a structural unit having several types of cells assembled in a certain pattern and which has a coherent role as a whole. Whether or not the cell aggregates are differentiated to the level of the biological tissue, is not limited. Further, whether or not the cell aggregates are constituted by matured cells, is not limited. The shape of the cell aggregates or the biological tissue is not limited.

The fluorinated copolymer to be used in the present embodiment contains from 5 to 99 mol% of units A based on CF₂=CFX (X is a fluorine atom, a chlorine atom, or CF₃). The fluorinated copolymer contains units B in addition to units A. The fluorinated copolymer may be either a random copolymer, a block copolymer or a graft copolymer.

To the total units in the fluorinated copolymer, units A are preferably from 10 to 90 mol%, more preferably from 80 to 20 mol%, with a view to achieving both the drug non-absorbability and transparency.

As units A, units based on tetrafluoroethylene (hereinafter referred to as TFE units), units based on hexafluoropropylene (hereinafter referred to as HFP units), or units based on chlorotrifluoroethylene (hereinafter referred to as CTFE units) may be mentioned.

The fluorinated copolymer preferably contains, as units B, at least one type selected from the group consisting of units based on propylene (hereinafter referred to as P units), units based on ethylene (hereinafter referred to as E units), units based on vinylidene fluoride (hereinafter referred to as VdF units), and units based on a perfluoro(alkyl vinyl ether) (hereinafter referred to as PAVE units).

The fluorinated copolymer to be used in the present embodiment is produced by a common radical polymerization method. In order to obtain high transparency, it is preferred that the composition distribution of the fluorinated copolymer is small, and as such a method, a method of reducing the variation of the material composition ratio in the polymerization by post-adding the respective raw materials depending on the reaction rates of the raw materials consumed during the polymerization, may be mentioned. Further, as the case requires, by using a living radical polymerization method such as an iodine transfer polymerization method of conducting radical polymerization in the presence of iodine alone or an iodine compound, it is possible to reduce the composition distribution of the fluorinated copolymer, whereby high transparency is obtainable.

Further, since the molecular weight distribution of the fluorinated copolymer will be reduced by the living radical polymerization method, it is possible to reduce the low molecular weight components that cause sticking to the mold, and thus, it is possible to reduce or eliminate coating the mold with a mold release agent that may adversely affect cells. In particular, in a case where a substrate of a chip having a narrow groove or small holes is to be prepared by a mold, if the mold releasability is poor, deformation or damage may be caused at the time of being peeled from the mold, and therefore, it is useful to employ a living radical polymerization method such as an iodine transfer polymerization method.

As preferred fluorinated copolymers, the following (1) to (6) may be exemplified.

(1) A copolymer (hereinafter referred to as TFE-P copolymer) having TFE units and P units, wherein to all units, the total of TFE units and P units is from 65 to100 mol%. (2) A copolymer (hereinafter referred to as TFE-E copolymer) having TFE units and E units, wherein to all units, the total of TFE units and E units is from 80 to 100 mol%. (3) A copolymer (hereinafter referred to as HFP-VdF copolymer) having HFP units and VdF units, wherein to all units, the total of HFP units and VdF units is from 50 to 100 mol%. (4) A copolymer (hereinafter referred to as CTFE-E copolymer) having CTFE units and E units, wherein to all units, the total of CTFE units and E units is from 80 to 100 mol%. (5) A copolymer (hereinafter referred to as TFE-PAVE copolymer) having TFE units and PAVE units, wherein to all units, the total of TFE units and PAVE units is from 50 to 100 mol%. (6) A copolymer (hereinafter referred to as TFE-HFP copolymer) having TFE units and HFP units, wherein to all units, the total of TFE units and HFP units is from 50 to 100 mol%.

(1) As the TFE-P copolymer, the following (1-1) or (1-2) may be exemplified.

(1-1) A copolymer wherein the total of TFE units and P units is from 65 to 100 mol%, and the molar ratio of TFE units/P units is from 30/70 to 70/30, preferably from 45/55 to 65/35, more preferably from 50/50 to 60/40. It may contain iodine atoms in an amount of from 0.01 to 5.0 mass%.

As units other than TFE units and P units, units based on the following other monomers may be exemplified.

Other monomers: a fluorinated olefin such as monofluoroethylene, trifluoroethylene, trifluoropropylene, pentafluoropropylene, hexafluoropropylene, hexafluoroisobutylene or dichlorodifluoroethylene; a hydrocarbon olefin such as ethylene, 1-butene or isobutylene; an alkyl vinyl ether such as methyl vinyl ether, ethyl vinyl ether, butyl vinyl ether or cyclohexyl vinyl ether; a vinyl ester such as vinyl acetate, or vinyl propionate; vinyl chloride, vinylidene chloride, and trifluorostyrene.

The content of other units is preferably at most 35 mol%, more preferably at most 33 mol%, further preferably at most 31 mol%, to all units.

The content of iodine atoms is preferably from 0.01 to 1.5 mass%, more preferably from 0.01 to 1.0 mass%, to the total mass of the copolymer.

(1-2) A copolymer comprising TFE units, P units and at least one type of units I derived from monomer I represented by the following formula (I), wherein the total of TFE units, P units and units I is from 98 to 100 mol%. It may contain iodine atoms in an amount of from 0.01 to 5.0 mass%.

CR¹R²=CR³-R⁴-CR⁵=CR⁶R⁷ (I)

(In the formula (I), R¹, R², R³, R⁵, R⁶ and R⁷ are each independently a hydrogen atom, a fluorine atom or a methyl group, and R⁴ is a perfluoroalkylene group having from 1 to 10 carbon atoms, or a group having an etheric oxygen atom at both ends, one end or between carbon-carbon atoms of the perfluoroalkylene group.)

As the monomer I, CF₂=CFO(CF₂)₃OCF=CF₂, CF₂=CFO(CF₂)₄OCF=CF₂, or CH₂=CH(CF₂)₆CH=CH₂ may be exemplified.

The content of units I to all units is preferably from 0.1 to 1.5 mol%, more preferably from 0.15 to 0.8 mol%, more preferably from 0.15 to 0.6 mol%.

The molar ratio of TFE units/P units is preferably from 30/70 to 99/1, more preferably from 30/70 to 70/30, further preferably from 40/60 to 60/40.

As units other than TFE units, P units and units I, units based on the following fluorinated monomer or non-fluorinated monomer may be exemplified.

As the fluorinated monomer, vinyl fluoride, pentafluoropropylene, perfluorocyclobutene, CH₂=CHCF₃, CH₂=CHCF₂CF₃, CH₂=CHCF₂CF₂CF₃, CH₂=CHCF₂CF₂CF₂CF₃ or CH₂=CHCF₂CF₂CF₂CF₂CF₃ may be exemplified.

As the non-fluorinated monomer, isobutylene, pentene, methyl vinyl ether, ethyl vinyl ether, propyl vinyl ether, butyl vinyl ether, vinyl acetate, vinyl propionate, vinyl butyrate, vinyl caproate or vinyl caprylate may be exemplified.

To all units, other units are preferably at most 2.0 mol%, more preferably at most 1.0 mol%, particularly preferably at most 0.5 mol%.

Further, to the total mass of the copolymer, iodine atoms are preferably from 0.01 to 1.5 mass%, more preferably from 0.01 to 1.0 mass%, from such a viewpoint that coloring can be suppressed.

(2) As the TFE-E copolymer, the following (2-1) or (2-2) may be exemplified.

(2-1) A copolymer wherein the total of TFE units and E units is from 80 to 100 mol%, and the molar ratio of TFE units/E units is from 75/25 to 30/70, preferably from 70/30 to 45/65, more preferably from 70/30 to 50/50. It may contain iodine atoms in an amount of from 0.01 to 5.0 mass%.

As units other than TFE units and E units, units based on monomers (2) to (7) as described in paragraph 0014 of WO2017/082417, may be exemplified.

In all units, other units are preferably at most 20 mol%, more preferably at most 15 mol%, further preferably at most 8 mol%.

(2-2) A copolymer comprising TFE units, E units and at least one type of units II derived from monomer II represented by the following formula (II), wherein the total of TFE units, E units and units II is 100 mol%. It may contain iodine atoms in an amount of from 0.01 to 5.0 mass%.

CH₂=CY¹(CF₂)ₙY² (II)

(In the formula (II), Y¹ and Y² are each independently a hydrogen atom or a fluorine atom, and n is an integer of from 2 to 8.)

As the monomer II, CH₂=CF(CF₂)ₙF, CH₂=CF(CF₂)ₙH, CH₂=CH(CF₂)ₙF or CH₂=CH(CF₂)ₙH may be exemplified. n is from 2 to 8, preferably from 3 to 7, more preferably from 4 to 6.

The content of units II to all units is preferably from 2.6 to 6.0 mol%, more preferably from 2.8 to 5.0 mol%, further preferably from 3.0 to 5.0 mol%, particularly preferably from 3.3 to 4.5 mol%.

The molar ratio of TFE units/E units is preferably from 51/49 to 60/40, more preferably from 52/48 to 57/43, further preferably from 53/47 to 55/45.

(3) As the HFP-VdF copolymer, the following (3-1) or (3-2) may be exemplified.

(3-1) A copolymer wherein the total of HFP units and VdF units is from 50 to 100 mol%, and the molar ratio of VdF units/HFP units is from 60/40 to 95/5, preferably from 70/30 to 90/10, more preferably from 75/25 to 85/15. It may contain iodine atoms in an amount of from 0.01 to 5.0 mass%.

As units other than HFP units and VdF units, units based on the following other monomers may be exemplified.

As other monomers, chlorotrifluoroethylene, trifluoroethylene, vinyl fluoride, ethylene, ethylidene norbornene or vinyl crotonate may be exemplified.

In all units, other units are preferably at most 50 mol%, more preferably at most 30 mol%, further preferably at most 10 mol%.

(3-2) A copolymer wherein the total of HFP units, VdF units and TFE units is from 50 to 100 mol%, and the molar ratio of VdF units/TFE units/HFP units is from 50/5/45 to 65/30/5, preferably from 50/15/35 to 65/25/10, more preferably from 50/20/30 to 65/20/15. It may contain iodine atoms in an amount of from 0.01 to 5.0 mass%.

As units other than HFP units, VdF units and TFE units, the above-mentioned units based on other monomers in the above (3-1) may be exemplified.

To all units, other units are preferably at most 50 mol%, more preferably at most 30 mol%, further preferably at most 10 mol%.

(4) As the CTFE-E copolymer, the following (4-1) may be exemplified.

(4-1) A copolymer wherein the total of CTFE units and E units is from 80 to 100 mol%, preferably from 85 to 100 mol%, further preferably from 90 to 100 mol%, and the molar ratio of E units/CTFE units is from 68/32 to 14/86, preferably from 55/45 to 35/65. It may contain iodine atoms in an amount of from 0.01 to 5.0 mass%.

As units other than CTFE units and E units, units based on monomers as described in paragraph 0025 of WO2014/168076 may be exemplified.

In all units, other units are preferably at most 20 mol%, more preferably at most 15 mol%.

(5) As the TFE-PAVE copolymer, the following (5-1), (5-2) or (5-3) may be exemplified.

(5-1) A copolymer in which the total of TFE units and PAVE units is from 50 to 100 mol%, and the molar ratio of TFE units/PAVE units is from 20/80 to 80/20, more preferably from 50/50 to 80/20. It may contain iodine atoms in an amount of from 0.01 to 5.0 mass%.

As the perfluoro(alkyl vinyl ether), perfluoro(methyl vinyl ether), perfluoro(ethyl vinyl ether), perfluoro(propyl vinyl ether), perfluoro(methoxyethyl vinyl ether), perfluoro(propoxyethyl vinyl ether) or perfluoro(propoxypropyl vinyl ether) may be exemplified.

As other units other than TFE units and PAVE units, the above-mentioned units based on other monomers in the above (3-1), HFP, and VdF, may be exemplified.

To all units, other units are preferably at most 50 mol%, more preferably at most 30 mol%, further preferably at most 10 mol%.

(5-2) A copolymer comprising TFE units, PAVE units and at least one type of units III derived from monomer III represented by the following formula (III), and the total of TFE units, PAVE units and units III is from 50 to 100 mol%. It may contain iodine atoms in an amount of from 0.01 to 5.0 mass%.

CR¹¹R¹²=CF-Q-R¹³-CO-Z (III)

(In the formula (III), R¹¹ and R¹² are each independently a hydrogen atom or a fluorine atom, Q is a single bond or an etheric oxygen atom, R¹³ is a fluoroalkylene group or a group having an etheric oxygen atom at least at one terminal or between carbon-carbon atoms of two or more fluoroalkylene groups, -Z is -OH, -OR¹⁴, -NR¹⁵R¹⁶, -NR¹⁷NR¹⁸H, or NR¹⁹OR²⁰, R¹⁴ is an alkyl group, R¹⁵, R¹⁶, R¹⁷, R¹⁸, R¹⁹ and R²⁰ are each independently a hydrogen atom or an alkyl group.)

In a case where R¹³ is a fluoroalkylene group, the number of carbon atoms is preferably from 1 to 6, more preferably from 1 to 4.

In a case where R¹³ is a fluoroalkylene group having an etheric oxygen atom, the number of carbon atoms is preferably from 2 to 10, more preferably from 2 to 6. The etheric oxygen atom is present at one terminal, between carbon-carbon atoms, or at both of them in the fluoroalkylene group.

In a case where R¹³ is a fluoroalkylene group having an etheric oxygen atom, a perfluoroalkylene group is preferred.

The number of carbon atoms in R¹⁴ is preferably from 1 to 6, more preferably 1 or 2.

R¹⁵, R¹⁶, R¹⁷, R¹⁸, R¹⁹ and R²⁰ are each independently preferably a hydrogen atom or an alkyl group having from 1 to 6 carbon atoms, more preferably a hydrogen atom or an alkyl group having 1 or 2 carbon atoms.

-Z is more preferably -OR¹⁴.

As the monomer III, CF₂=CFO(CF₂)₂COOCH₃, CF₂=CFO(CF₂)₃COOCH₃, CF₂=CFO(CF₂)₄COOCH₃, CF₂=CFO(CF₂)₅COOCH₃, CF₂=CFOCF₂CF(CF₃)O(CF₂)₂COOCH₃, CF₂=CFOCF₂CF(CF₃)O(CF₂)₃COOCH₃, CF₂=CFO(CF₂)₂O(CF₂)₂COOCH₃, CF₂=CFO(CF₂)₃O(CF₂)₂COOCH₃, CH₂=CFCF₂OCF(CF₃)COOCH₃, or CH₂=CFCF₂OCF(CF₃)CF₂OCF(CF₃)COOCH₃
may be exemplified.

By irradiation with light having a wavelength of from 150 to 300 nm, -Q-R¹³-CO-Z in units III undergoes a reaction to release -CO-Z, thereby to form -Q-R¹³ · radicals, and two -Q-R¹³ · radicals are reacted to form an intermolecular cross-linking structure (-Q-R¹³-R¹³-Q-).

The content of units III to all units is preferably from 0.01 to 100 mol%, more preferably from 0.1 to 20 mol%, further preferably from 0.1 to 5 mol%.

The molar ratio of TFE units/PAVE units is preferably from 5/95 to 95/5, more preferably from 20/80 to 80/20, further preferably from 50/50 to 70/30.

As other units other than TFE units, PAVE units and the monomer III, units (4) as described in paragraph 0041 of WO2015/098773 may be exemplified.

In all units, other units are preferably at most 30 mol%, more preferably at most 10 mol%.

Per 1 g of the fluorinated polymer of the present embodiment, the content of the group represented by -CO-Z is preferably from 0.1 to 4 mmol/g, more preferably from 0.1 to 3 mmol/g, further preferably from 0.3 to 1 mmol/g.

In the case of molding the fluorinated polymer of this embodiment into a film, the weight average molecular weight of the fluorinated polymer as converted to polymethyl methacrylate, as calculated by size exclusion chromatogram, is preferably from 1,000 to 1,000,000, more preferably from 5,000 to 300,000, further preferably from 10,000 to 50,000. The value of the weight average molecular weight/the number average molecular weight is preferably from 1 to 20, more preferably from 1 to 10, further preferably from 1 to 3.

(5-3) A copolymer wherein the total of TFE units and PAVE units is from 50 to 100 mol%, and the molar ratio of TFE units/PAVE units is from 92/8 to 99/1, more preferably from 95/5 to 99/1. It may contain iodine atoms in an amount of from 0.01 to 5.0 mass%.

As other units other than TFE units and PAVE units, the units based on other units in the above (3-1), HFP and VdF may be exemplified.

To all units, other units are preferably at most 10 mol%, more preferably at most 5 mol%.

(6) As the TFE-HFP copolymer, the following (6-1) may be exemplified.

(6-1) A copolymer wherein the total of TFE units and HFP units is from 50 to 100 mol%, and the molar ratio of TFE units/HFP units is from 96/4 to 87/13, preferably from 95/5 to 85/15. It may contain iodine atoms in an amount of from 0.01 to 5.0 mass%.

As other units other than TFE units and HFP units, the units based on other monomers in the above (3-1) and VdF may be exemplified.

To all units, other units are preferably at most 10 mol%, more preferably at most 5 mol%.

Among the above-mentioned fluorinated copolymers, preferred from the viewpoint of transparency is the TFE-P copolymer, the TFE-E copolymer, the CTFE-E copolymer, or the TFE-PAVE copolymer, and a TFE-PAVE copolymer is particularly preferred.

Preferred from the viewpoint of flexibility is the TFE-P copolymer, the HFP-VdF copolymer, or the TFE-PAVE copolymer, and the HFP-VdF copolymer is particularly preferred.

Further, in a case where the fluid flowing through the flow channel is composed mainly of water, as the difference in refractive index between the fluorinated copolymer and water is smaller, scattering and refraction of light at the interface between the channel and the fluid will be suppressed, and inside of the flow channel can be easily observed, and thus, the TFE-P copolymer, the TFE-E copolymer, the HFP-VdF copolymer, or the TFE-PAVE copolymer, is preferred, and the TFE-PAVE copolymer is particularly preferred.

A film made of the fluorinated copolymer to be used in this embodiment can be produced by a method of molding by means of a mold by using the fluorinated copolymer alone or by preparing a fluorinated copolymer composition containing the fluorinated copolymer. For example, it can be produced by a method wherein the fluorinated copolymer or the fluorinated copolymer composition is made flowable by heating as the case requires, and then poured into a mold under pressure as the case requires, or applied to the mold surface, followed by curing. The material for the mold is not limited. For example, a mold made of a metal (also referred to as a metal mold), a mold made of a silicon substrate and a resist resin using photolithography, or a mold having the shape transferred by using an epoxy resin and the like from the above mold or molded product, may be mentioned. As the curing method, a method of cooling or a method of crosslinking by ultraviolet irradiation, radiation irradiation, electron beam irradiation or heating, may be exemplified. The degree of curing of the film may be such a degree that it does not flow at the time of use of the chip. The film may be processed, as the case requires, by drilling, by a machining method using a blade or laser, or by a micro blasting method.

The fluorinated copolymer composition may contain a solvent and additives as the case requires.

Among the above-mentioned fluorinated copolymers, particularly preferred from such a viewpoint that it will be easily molded into a film, is the TFE-P copolymer, the TFE-E copolymer, the CTFE-E copolymer, or the TFE-PAVE copolymer, and the TFE-P copolymer or the TFE-E copolymer is particularly preferred.

Further, the TFE-P copolymer or the above (5-1) is preferably used as mixed and vulcanized with a known crosslinking agent, a crosslinking aid, etc., as the case requires, for e.g. the physical properties as a rubber.

In the chip of this embodiment, two openings and an inner space connecting the openings are formed, and it is possible to culture cells in the inner space. Further, the surface in contact with the inner space is made of a fluorinated copolymer and is excellent in drug non-absorbability. Particularly, as the drug, it is excellent in non-absorbability of a hydrophobic low molecular compound. It is excellent in non-absorbability of a hydrophobic low molecular compound having an octanol/water partition coefficient logP of at least 0, particularly preferably at least 3, as an index for hydrophobicity. Thus, the chip of this embodiment is suitable as a culture vessel to conduct an evaluation test of various drugs using cells, and it is possible to improve the accuracy of the test. As the case requires, at least on a surface which becomes to be a storage portion for cells on the surface in contact with the inner space, a protein layer such as fibronectin or its gel, collagen or its gel, or gelatin or its gel may be provided.

Further, a film suitable for cell culture, for example, a porous member having pores with such a pore size that the object, such as cells, to be observed or measured, will not flow, may be provided in the inner space and may be used as a storage portion, and the object may be placed thereon. As the porous member, a membrane made of collagen such as Vitrigel (Vitrigel, as defined at pages 463 to 473 in Cell Transplantation 2004, Vol. 13, No. 4), a porous membrane prepared from polydimethylsiloxane, such as Sylgard (Dow Corning trade name), a porous membrane made of polyethylene terephthalate, such as Falcon (Corning trade name), a honeycomb structure film made of a polymer which is soluble in a hydrophobic solvent, which is prepared by a technique of JP-A-2002-335949, a stretched polytetrafluoroethylene porous membrane, such as VECELL (Vessel Inc. trade name), a polyurethane nonwoven fabric such as Espansione (KB Seiren, Ltd. trade name), etc. may be mentioned.

In the present embodiment, the types of the fluorinated copolymers which respectively constitute the first to third substrates, may be the same or different. In particular, it is preferred to form the entire chip by one type of the fluorinated copolymer, since uniformity will be excellent, whereby scattering or refraction of light will be suppressed, and it will be easier to observe cells.

The fluorinated copolymer is preferably excellent in gas permeability, since it is thereby possible to properly conduct e.g. culturing of cells without presenting a load on the cells when used in the chip. The gas permeability can be evaluated by a transmission coefficient of a gas measured by using a gas permeation test-difference pressure method of "JIS K7126-1: 2006". In the present invention, the oxygen transmission coefficient of the fluorinated copolymer is preferably from 1.0 to 390×10⁻¹⁰ [cm³·cm/(cm²·s·cmHg), in the following, this unit may be omitted], more preferably from 1.0 to 100×10⁻¹⁰. The carbon dioxide transmission coefficient is preferably from 5.0 to 1,900×10⁻¹⁰ [cm³·cm/(cm²·s·cmHg), in the following, this unit may be omitted], more preferably from 5.0 to 1,000×10⁻¹⁰.

Further, the permeability of water vapor can be evaluated by a transmission coefficient which is measured by using a water vapor permeation test-MOCON method of "JIS K7129: 2008". In the present invention, the water vapor transmission coefficient of the fluorinated copolymer is preferably from 0.1 to 18 [g·mm/(m²·24h), in the following, this unit may be omitted], more preferably from 0.1 to 2. When the respective transmission coefficients are within these ranges, the gas permeability will be good, and the volatilization of liquid such as water in the inner space of the chip can be suppressed, whereby it can be suitably used as a chip.

As such a fluorinated copolymer, a copolymer containing TFE units in an amount of from 50 to 80 mol% is preferred. Especially, the TFE-P copolymer or the TFE-PAVE copolymer is preferred, and the copolymer of the above (1-2) or the above (5-2) is particularly preferred.

The above fluorinated copolymer is preferably one having the following hardness and Young's modulus, because it has flexibility and it is possible to impart a proper mechanical stretch to cells when used in the chip. The hardness is preferably a Type A durometer hardness of at most 80, more preferably at most 60. The Young's modulus is preferably at most 10 MPa, more preferably at most 5.0 MPa, particularly preferably at most 3.0 MPa.

The fluorinated copolymer is also preferably excellent in non-absorbability of a specific drug, since when used in a chip, it is less likely to absorb the drug, and various evaluation tests can be conducted properly. For example, it is preferably excellent in non-absorbability of nifedipine being a hypertension and angina pectoris therapeutic agent, or Bay K8644 having a calcium channel inhibitory action. As a method for measuring non-absorbability, a method may be mentioned which is described in reference document (Biochemical and Biophysical Research Communications 482 (2017) 323-328). The nifedipine absorption rate is preferably at most 30%, more preferably at most 20%, particularly preferably at most 10%. The absorption rate for Bay K8644 is preferably at most 10%, more preferably at most 5%, particularly preferably at most 2%.

If the flexibility is improved by lowering the hardness, the fluorinated copolymer tends to readily absorb the drug. Further, to be used in a chip, it is required to have transparency so that the inner space of the chip can be visually observed. In order to have the hardness, the Young's modulus, the drug non-absorbability and the transparency as combined, the fluorinated copolymer is preferably a copolymer containing TFE units in an amount of from 50 to 70 mol%. Especially, the TFE-P copolymer or the TFE-PAVE copolymer is preferred, and a copolymer of the above (1-2) or the above (5-2) is particularly preferred.

### <Second embodiment>

The second embodiment of the chip of the present invention is one obtained by coating a fluorinated copolymer to the whole of the surface in contact with the inner space S of the chip having a shape shown in Fig. 1. Although not shown, a coating layer of a fluorinated copolymer is present on the entire surface of the inner wall surface 1a of the through hole S1, the inner wall surface 1b of the through hole S2, the surface 1c facing the through groove S3 of the first substrate 1, the inner wall surface 2b of the through groove S3, and the surface 3a facing the through groove S3 of the third substrate 3.

The first to third substrates and the coating layer are preferably transparent from such a viewpoint that it will be easy to observe the inside, and the preferred embodiment is similar to the first embodiment. Further, also with respect to hardness, the same as the first embodiment will apply.

The thickness of the coating layer is preferably from 0.1 to 200 µm, more preferably from 1 to 50 µm. When the thickness of the coating layer is at least the lower limit value in the above range, it will be excellent in drug non-absorbability. When the thickness is at most the upper limit value, it will be easy to maintain the shape of the flow channel.

As the fluorinated copolymer, the same one as in the first embodiment may be employed. In this embodiment, a fluorinated copolymer composition comprising a fluorinated copolymer and a solvent, is prepared and used as a coating liquid. The fluorinated copolymer composition may contain additives as the case requires. As the additives, a perfluoropolyether, etc. may be mentioned.

Among the above-mentioned fluorinated copolymers, preferred to form the coating layer is the TFE-P copolymer, the HFP-VdF copolymer, the CTFE-E copolymer, or the TFE-PAVE copolymer, and the TFE-P copolymer or the TFE-PAVE copolymer is particularly preferred.

The material for the chip is not limited. A material which can be molded to form a transparent film, is preferred. A material having flexibility is preferred.

As the material for the chip, a fluorinated copolymer different from the coating layer, a polysiloxane-type polymer (polydimethylsiloxane, diphenylsiloxane, etc.), a silicone resin, a silicone rubber, a natural rubber, an isoprene rubber, a butadiene rubber, a chloroprene rubber, a polyolefin, an acrylic resin, an acrylic rubber, a styrene resin, a styrene rubber, a polyester, a polycarbonate, a polyurethane, a polyvinyl chloride, an epichlorohydrin rubber, a polyvinyl alcohol, a polylactic acid, a polyglycolic acid, a thermoplastic elastomer, or silica glass may be exemplified.

The chip of this embodiment can be produced, for example, by the following method.

Three sheets of transparent film are prepared and processed, laminated and bonded in the same manner as for the chip of the first embodiment, to obtain a main body. Then, the coating solution is applied to the surface in contact with the inner space in the body, to form a coating layer of a fluorinated copolymer. Or, before laminating the three films, a coating layer of a fluorinated copolymer may be formed at the portion to become the surface in contact with the inner space, in each film. For example, first, three transparent films are prepared, and processed in the same manner as for the chip of the first embodiment. On the inner wall surface 1a of the through hole S1, the inner wall surface 1b of the through hole S2 and the surface 1c facing the through groove S3, of the film which becomes to be the first substrate 1, a coating liquid is applied to form a coating layer of a fluorinated copolymer. On the inner wall surface 2b of the through groove S3 of the film which becomes to be the second substrate 2, a coating liquid is applied to form a coating layer of a fluorinated copolymer.

On the surface 3a facing the through groove S3 of the film which becomes to be the third substrate 3, a coating liquid is applied to form a coating layer of a fluorinated copolymer. Thereafter, these three films were laminated and bonded in the same manner as in the first embodiment, to obtain a chip of this embodiment.

Also in this embodiment, the same effects as in the first embodiment can be obtained. In this embodiment, as compared with the first embodiment, the degree of freedom of the material for the main body is high, and thus, it is possible to further reduce the cost.

### <Third embodiment>

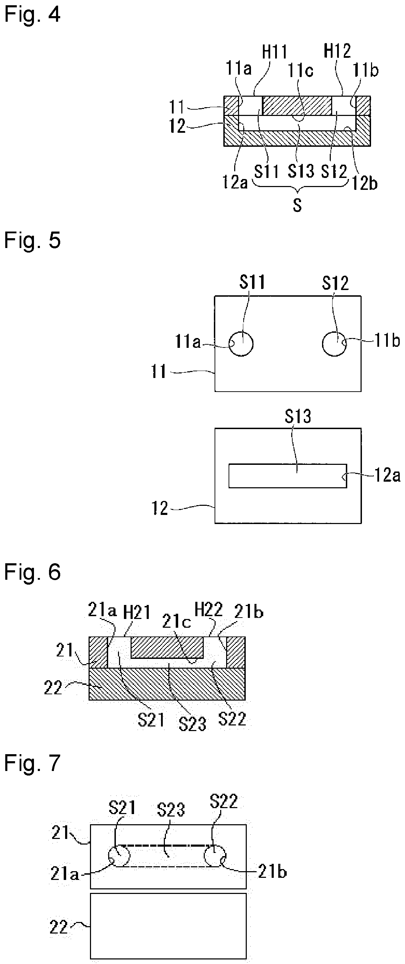

Fig. 4 is a schematic cross-sectional view showing a third embodiment of the chip of the present invention, and Fig. 5 is a schematic plan view wherein the chip is disassembled to the first substrate and the second substrate. The points where this embodiment is different from the first embodiment, are that while the chip of the first embodiment was a laminate of three substrates, the chip of this embodiment is a laminate of two substrates, and the third substrate 3 is not used, and that while in the first embodiment, the second substrate 2 was provided with a through groove S3, in the present embodiment, a bottomed groove S13 is formed in the second substrate 12. In this embodiment, the first substrate and the second substrate are made of a fluorinated copolymer.

In the chip of this embodiment, the first substrate 11 and the second substrate 12 are laminated. In the first substrate 11, two through holes S11, S12 are formed. The open ends on the side opposite to the second substrate 12, of the through holes S11, S12 are openings H11, H12. The symbol 11a denotes the inner wall surface of the through hole S11, and 11b denotes the inner wall surface of the through hole S12.

In the second substrate 12, a bottomed groove S13 is provided. The bottomed groove S13 is provided so that when the first substrate 11 and the second substrate 12 are laminated, the bottomed groove S13 connects the through holes S11, S12. The symbol 11c denotes a surface facing the bottomed groove S13, of the first substrate 11, and 12b denotes a bottom surface of the bottomed groove S13.

In the chip in which the first substrate 11 and the second substrate 12 are laminated, the space formed by the through holes S11, S12 and the bottomed groove S13 is the inner space S to connect the openings H11, H12.

The chip of this embodiment can be used as a vessel for culturing cells in the inner space S. In that case, the inner space S includes a storage portion (not shown) for storing cells. For example, a storage portion (not shown) to store cells may be provided on the bottom surface 12b of the bottomed groove S13.

The chip of this embodiment can be produced, for example, by the following method.

Two films made of a fluorinated copolymer are prepared and used as the first and second substrates. Two through holes S11, S12 penetrating from the front surface to the back surface are provided in the first substrate 11. In the second substrate 12, a bottomed groove S13 is provided.

So that the bottomed groove S13 connects the the two through holes S11, S12, the first substrate 11 and the second substrate 12 are laminated, and the substrates are liquid-tightly bonded to each other to obtain a chip.

### <Fourth embodiment>

The point where this embodiment is different from the second embodiment is that a fluorinated copolymer is coated on the whole of the surface in contact with the inner space S of the chip having a shape as shown in Fig. 4. Although not shown, a covering layer of a fluorinated copolymer is present on the entire surface of the inner wall surface 11a of the through hole S11, the inner wall surface 11b of the through hole S12, the surface 11c facing the bottomed groove S13 of the first substrate 11, the inner wall surface 12a of the bottomed groove S13, and the bottom surface 12b of the bottomed groove S13.

### <Fifth embodiment>

Fig. 6 is a schematic cross-sectional view showing a fifth embodiment of the chip of the present invention. Fig. 7 is a schematic plan view wherein the chip is disassembled to the first substrate and the second substrate. The points where this embodiment is different from the third embodiment are that while the second substrate of the third embodiment has a bottomed groove, the second substrate surface of this embodiment is flat at the front surface and the back surface, and that the first substrate of the third embodiment has, not only the through holes, but also a bottomed groove so as to connect the through holes. Preferred embodiments of the respective substrates and the production method are similar to the first and third embodiments.

### <Sixth embodiment>

The sixth embodiment of the chip of the present invention is one obtained by coating a fluorinated copolymer to the whole of the surface in contact with the inner space of the main body having a shape as shown in Fig. 6. Preferred embodiments of the respective substrates, the coating method and the production method are similar to the second embodiment and the fourth embodiment.

### <Seventh embodiment>

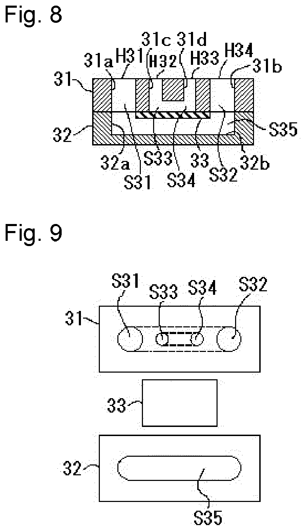

Fig. 8 is a schematic cross-sectional view showing a seventh embodiment of the chip of the present invention, and Fig. 9 is a schematic plan view wherein this chip was disassembled to the first substrate and the second substrate. The points where this embodiment is different from the fifth embodiment are that while the second substrate of the fifth embodiment is flat, the second substrate of this embodiment has a bottomed groove disposed so as to connect the through holes, and that it has, between the first substrate and the second substrate, a film disposed so as not to block the through holes. Preferred embodiments of the respective substrates, and the production method are similar to the first embodiment, the third embodiment and the fifth embodiment.

### <Eighth embodiment>

An eighth embodiment of the chip of the present invention is one obtained by coating a fluorinated copolymer to the whole of the surface in contact with the inner space of the chip having a shape as shown in Fig. 8. Preferred embodiments of the substrates, the coating method and the production method are similar to the second embodiment, the fourth embodiment and the sixth embodiment.

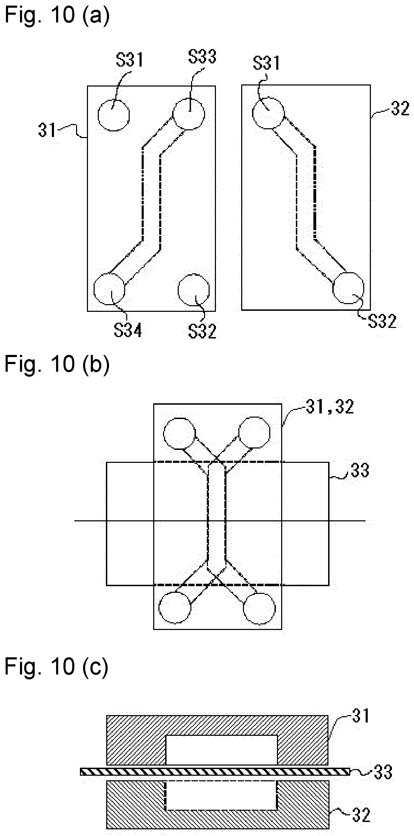

Fig. 10 shows specific structures of the seventh and eighth embodiments. The inner space of the first substrate and the inner space of the second substrate are independently present up and down, whereby different drugs can be flown into the chip without being mixed.

The left view and the right view in Fig. 10 (a) are, respectively, plan views of the first substrate and the second substrate. Fig. 10 (b) is a plan view of the state in which the first substrate and the second substrate are laminated. Fig. 10 (c) is a cross-sectional view of the state in which the first substrate and the second substrate are laminated.

### <Modification>

In the first to eighth embodiments, whole of the surface in contact with the inner space of the chip is made to be a surface made of a fluorinated copolymer, but it is possible to make at least a part of the surface in contact with the inner space to be a surface made of a fluorinated copolymer. By making a part of the surface in contact with a drug in the chip, to be a surface made of a fluorinated copolymer, it is possible to obtain an effect of reducing the drug to be absorbed in the chip. From the viewpoint of being more excellent in drug non-absorbability, it is more preferred that whole of the surface in contact with the drug in the chip is a surface made of a fluorinated copolymer.

In a case where only a part of the surface in contact with the inner space of the chip is a surface made of a fluorinated copolymer, it is preferred that the rest of the surface is a surface (exposed surface) where the substrate made of the following another material is exposed.

As another material, from such a viewpoint that absorption of a drug is less, silicate glass, stainless steel, cycloolefin polymer, polytetrafluoroethylene, or polychlorotrifluoroethylene is preferred. From the viewpoint of transparency, silicate glass or cycloolefin polymer is more preferred.

For example, in the first embodiment, the first substrate 1 and the third substrate 3 may be made of silicate glass, and the second substrate 2 may be composed of a fluorinated copolymer as in the first embodiment.

A substrate made of silicate glass is available from commercial sources.

In a method for producing the chip of this embodiment, first, through holes S1, S2 are formed in the glass film to become the first substrate 1. In the second substrate 2, a through groove S3 is formed as in the first embodiment. A glass film to become the third substrate 3 is not processed. Then, these three films are laminated and bonded to obtain a chip.

For example, in the second embodiment, the first substrate 1 and the third substrate 3 may be made of silicate glass, the second substrate 2 may be made of polysiloxane polymer, and a coating layer of a fluorinated copolymer may be provided only on the inner wall surface 2b of the through-groove S3 in the second substrate 2.

In the method for producing the chip of this embodiment, first, through holes S1, S2 are formed in the glass film to become the first substrate 1. In the second substrate 2, a through groove S3 is formed as in the second embodiment, and a coating layer of a fluorinated copolymer is formed by applying a coating liquid on its inner wall surface 2b. A glass film to become the third substrate 3 is not processed. Then, these three films are laminated and bonded to obtain a chip.

For example, in the third embodiment, the first substrate 11 may be made of silica glass, and the second substrate 12 may be made of a fluorinated copolymer as in the third embodiment.

In the method for producing a chip of this embodiment, first, through holes S11, S12 are formed in the glass film to become the first substrate 11. In the second substrate 12, a bottomed groove S13 is formed as in the third embodiment. Then these two films are laminated and bonded to obtain a chip.

For example, in the fourth embodiment, the first substrate 11 may be made of silica glass, the second substrate 12 may be made of polysiloxane polymer, and a coating layer of a fluorinated copolymer may be formed only on the inner wall surface 12a of the bottomed groove S13 in the second substrate 12 and the bottom surface 12b of the bottomed groove S13.

In the method for producing a chip of this embodiment, first, through holes S11, S12 are formed in a glass film to become the first substrate 11. In the second substrate 12, a bottomed groove S13 is formed in the same manner as in the fourth embodiment, and a coating layer of a fluorinated copolymer is formed by applying a coating liquid on its inner wall surface 12a and bottom surface 12b. Then, these two films are laminated and bonded to obtain a chip.

As a method for forming through holes in the substrate made of silicate glass, a processing method by laser, micro blasting or etching may be exemplified.

In the case of using substrates made of silica glass, as a method of bonding the substrates to one another, a method of overlaying substrates one on another and further crosslinking them by UV irradiation, radiation irradiation, electron beam irradiation or heating, a method of providing an adhesive layer between the substrates, a method of activating the bonding surface by corona discharge irradiation, plasma irradiation, ultraviolet irradiation, ozone exposure, base exposure, etc., followed by lamination, and a method of having two or more of these methods combined, may be exemplified.

In the first to eighth embodiments, two openings are formed, but the number of openings is not limited so long as it is plural. One inner space may connect three or more openings. The shape of the openings may be of any optional shape. Openings are not limited to be formed on the upper surface of the chip and may be provided on the lower surface or side surface, or these may be combined.

In the first and second embodiments, the surface on the second substrate side, of the third substrate, is made to be a flat surface, but on this surface, a bottomed groove may be formed. Or, in the third substrate, a through groove may be formed, and on the side opposite to the second substrate, a fourth substrate may be provided. The surface on the third substrate side, of the fourth substrate, may be a flat surface, or may have a bottomed groove formed.

For example, a chip having a structure as disclosed in a non-patent document (edited by Dongeun Huh, et al, "Microfabrication of human organs-on-chips", NATURE PROTOCOLS, vol.8, no.11, 2013, pp. 2,135 to 2,157) can be produced by a fluorinated copolymer.

Further, by the method as described in the above non-patent document, cells can be cultured in the chip.

In the case of culturing cells in the chip, as mentioned above, the chip may be deformed as the case requires, to impart mechanical strength to the cells in the inner space, and give mechanical stretch that mimics the motion of an organ. At that time, in order to give mechanical stretch, elongation of the cell culture surface is required, but if the elongation is too much, the cells will die by not withstanding the deformation. In the state of containing cells in the inner space of the chip, the elongation of the cell culture surface is preferably from 1.0 to 25.0%, more preferably from 1.0 to 10.0%, particularly preferably from 1.0 to 7.0%.

As such a fluorinated copolymer, a copolymer containing TFE units in an amount of from 50 to 70 mol% is preferred. Especially, the TFE-P copolymer is preferred, and the copolymer of the above (1-2) is particularly preferred.

Further, in the case of cultured cells, it is preferred that cell adhesiveness in the inner space is good. Especially, the TFE-P copolymer or the TFE-PAVE copolymer is preferred, the copolymer of the above (1-2) or the above (5-2) is more preferred, and the copolymer of the above (5-2) is particularly preferred. Further, with the above (1-2), it is possible to improve cell adhesion by carrying out appropriate crosslinking or surface treatment.

A method for preparing a substrate having a desired shape may be a method of using a 3D printer.

Further, by using a 3D printer, a chip may be prepared wherein the entire inner space is formed in one substrate.

### EXAMPLES

In the following, the present invention will be described in more detail with reference to Examples, but the present invention is not limited to these Examples.

### <Evaluation method for drug non-absorbability>

### [Preparation of 1.0 µM Nile red aqueous solution]

Nile red (manufactured by Tokyo Kasei Kogyo Co., Ltd.) was dissolved in ethanol, and further ion-exchanged water was added to prepare a 1.0 µM Nile red aqueous solution (containing 0.03 mass% of ethanol).

### [Preparation of test film]

A film to be evaluated was immersed in the 1.0 µM Nile red aqueous solution at room temperature (25°C) for 1 hour, then transferred to a container containing ion-exchanged water and stirred for 1 minute, whereupon water droplets on the surface of the taken out film were blown off with nitrogen gas to obtain a test film.

### [Cross-sectional observation by fluorescence microscope]

The test film was put in a freezing embedding medium (manufactured by Sakura Finetek) and frozen at -20°C by using a cryostat (manufactured by Leica, Inc.), followed by cutting to obtain a frozen section of 60 µm in thickness. Using an adhesive tape, the obtained frozen section was attached to slide glass, whereupon using a fluorescence microscope system (manufactured by Thermo Fisher Scientific, product name, EVOS, FL Auto), the fluorescence image (red) and the phase image of the cross-section of the sample were observed. The fluorescence microscope system was set to be Light = 35/100, exposure time = 320 ms, and GAIN = 12.0 db.

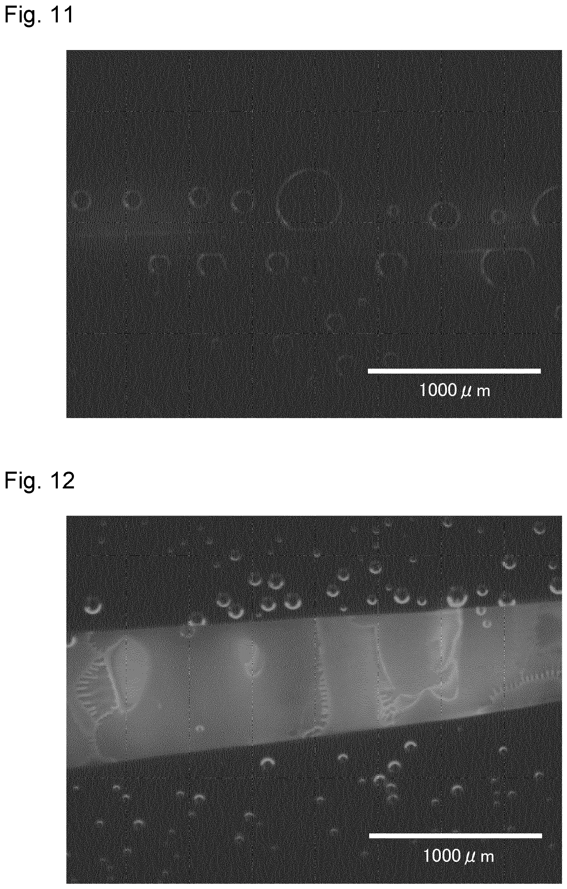

In the photographs of fluorescence images shown in Figs. 11 to 18, a white portion is a portion where a red fluorescence was observed. The white portion being smaller means that the absorption of the drug (1.0 µM Nile red aqueous solution) is less.

### (Experimental Example 1)

In the same procedure as in Production Example 3 in WO2015/098773, fluorinated copolymer P1 comprising 1.2 mol% of units based on CF₂=CFOCF₂CF₂CF₂COOCH₃, 25.4 mol% of units based on CF₂=CFOCF₂CF₂CF₃ and 73.5 mol% of units based on tetrafluoroethylene, was obtained. The weight average molecular weight as converted to polymethyl methacrylate as calculated by a size exclusion chromatogram, was 100,000, and the value of the weight average molecular weight/the number average molecular weight was 1.5.

The fluorinated copolymer P1 was formed into a film of 100 µm in thickness by hot pressing and placed in a container having a synthetic quartz plate of 3 mm in thickness at the irradiation surface, whereupon under a nitrogen atmosphere, using a 200 W low pressure mercury lamp (manufactured by Sen Lights Corporation, EUV200GL-31 ozone-less type, wavelength center: 254 nm) ultraviolet irradiation was conducted for 1 hour, to obtain a colorless transparent fluorinated crosslinked film Q1. The distance from the film to the lamp was 25 mm.

By the above-described method, the drug non-absorbability of the fluorinated crosslinked film Q1 was evaluated. The results are shown in Fig. 11. Fluorescence derived from the Nile red was not observed in the interior of the film. The test film remained to be colorless transparent even by visual inspection.

### (Comparative Example 1)

The drug non-absorbability of a colorless transparent polydimethylsiloxane crosslinked film (manufactured by Dow Corning, tradename, Sylgard, 184) Q2 was evaluated. The results are shown in Fig. 12. Fluorescence derived from the Nile red was observed in the interior of the film of 500 µm in thickness. The test film was colored in red as visually observed.

### (Experimental Example 2)

The fluorinated copolymer P1 in Experimental Example 1 was dissolved in n-C₆F₁₃H (AGC trade name, ASAHIKLIN AC-2000) to obtain a 10 mass% fluorinated copolymer P1 solution.

Coating was carried out by repeating an operation of immersing the polydimethylsiloxane crosslinked film Q2 in Comparative Example 1 in the fluorinated copolymer P1 solution, followed by pulling it up, 5 times. Then, it was left to stand at room temperature (25°C) for 2 hours for drying, whereupon under the same conditions as Experimental Example 1, both sides were subjected to UV irradiation, for one hour each, to obtain a colorless transparent fluorinated copolymer coating film Q3.

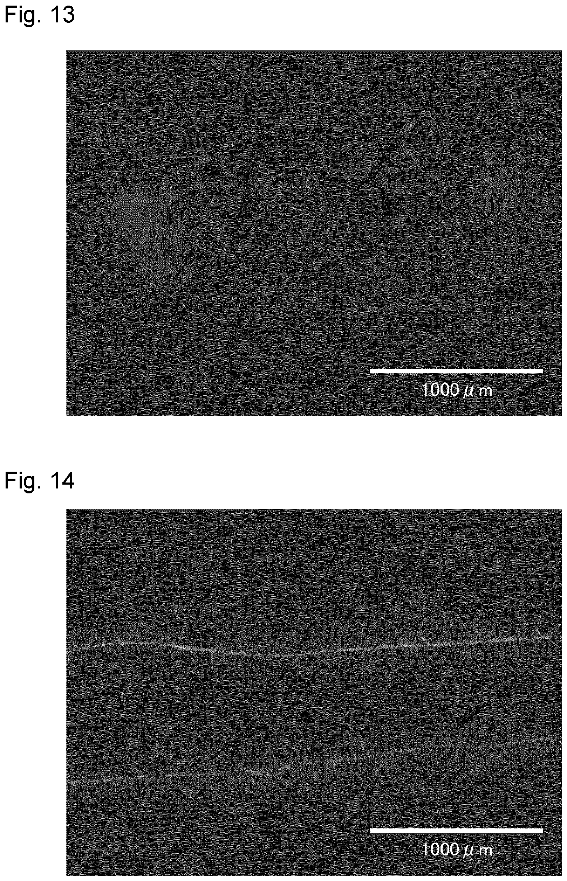

The evaluation results of the drug non-absorbability of the fluorinated copolymer coating film Q3 are shown in Fig. 13. Fluorescence derived from the Nile red was not observed in the interior of the film. The test film remained to be colorless transparent even by visual inspection. The thickness at the thinnest portion of the coating layer of the fluorinated copolymer P1 as measured from the phase image, was 12 µm.

### (Experimental Example 3)

In accordance with Example in Japanese Patent No. 5321580, fluorinated copolymer P2 comprising 56 mol% of TFE units and 44 mol% of P units, was synthesized.

The fluorinated copolymer P2 was dissolved in a mixed solvent comprising 40 mass% of 1,2,4-trimethylbenzene and 60 mass% of CF₃CH₂OCF₂CF₂H (AGC trade name, ASAHIKLIN AE-3000) to obtain a 10 mass% fluorinated copolymer P2 solution.

Coating was carried out by repeating an operation of immersing the polydimethylsiloxane crosslinked film Q2 in Comparative Example 1 in the fluorinated copolymer P2 solution, followed by pulling it up, three times. Then, it was heated and dried in a 170°C air-circulating oven for 1 hour to obtain a colorless transparent fluorinated copolymer coating film Q4.

The evaluation results of the drug non-absorbability of the fluorinated copolymer coating film Q4 are shown in Fig. 14. Fluorescence derived from the Nile red was not observed in the interior of the film. The test film remained to be colorless transparent even by visual inspection. The thickness of the thinnest portion of the coating layer of the fluorinated copolymer P2 as measured from the phase image, was 22 µm.

### (Experimental Example 4)

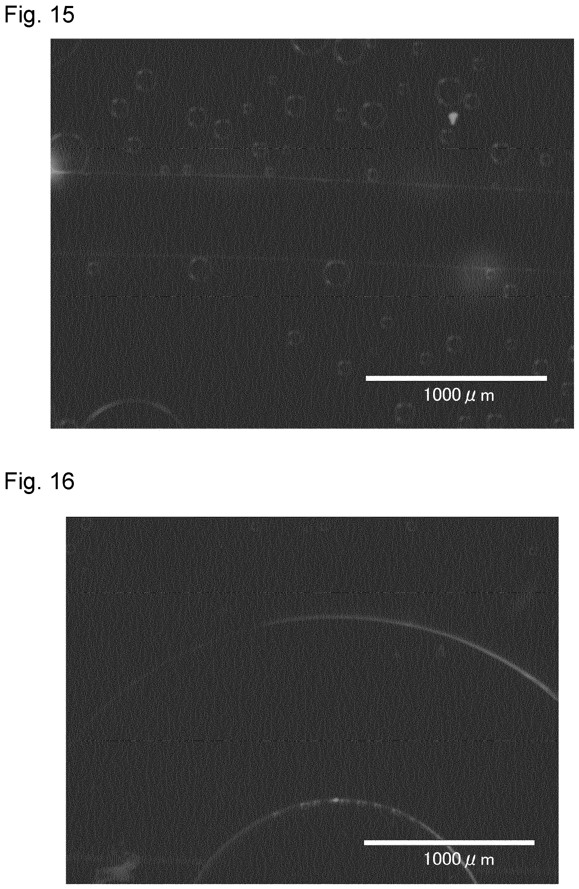

The evaluation results of drug non-absorbability of a colorless transparent 550 µm thick film Q5 of copolymer P3 (Daikin Industries, Ltd. trade name, DAI-EL G-901) comprising VdF units, TFE units and HFP units, are shown in Fig. 15. Fluorescence derived from the Nile red was not observed in the interior of the film. The test film remained to be colorless transparent even by visual inspection.

### (Experimental Example 5)

The evaluation results of drug non-absorbability of a colorless transparent 1.0 mm thick tube Q6 of a fluorinated thermoplastic elastomer P4 (Tigers Polymer Corporation product name: Tiger Flon) being a block copolymer of a TFE-E copolymer and HFP-VdF copolymer, are shown in Fig. 16. Fluorescence derived from the Nile red was not observed in the interior of the tube. The test film remained to be colorless transparent even by visual inspection.

### (Experimental Example 6)

In accordance with Example 2 in WO2017/082417, fluorinated copolymer P5 comprising 47.3 mol% of E units and 52.7 mol% of TFE units was synthesized.

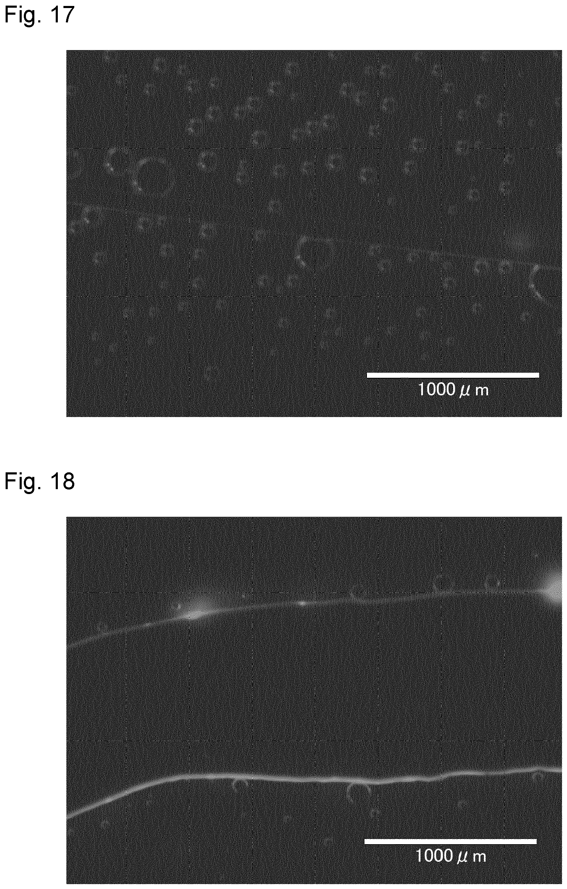

The fluorinated copolymer P5 was hot pressed to obtain a colorless transparent fluorinated copolymer film Q7 of 100 µm in thickness. The results of drug non-absorbability of the fluorinated copolymer film Q7 are shown in Fig. 17. Fluorescence derived from the Nile red was not observed in the interior of the film. The test film remained to be colorless transparent even by visual inspection.

### (Experimental Example 7)

In accordance with Example 5 of WO2017/057512, fluorinated copolymer P6 comprising 56 mol% of TFE units, 43.8 mol% of P units and 0.2 mol% of units based on CF₂=CFO(CF₂)₃OCF=CF₂ and containing 0.5 mass% of iodine, was synthesized.

The fluorinated copolymer P6 was hot-pressed to obtain a colorless transparent fluorinated copolymer film Q8 of 560 µm in thickness. The fluorinated copolymer film Q8 was measured by a haze meter in accordance with JIS-K-7136, whereby the haze value was 10%. The results of drug non-absorbability of the fluorinated copolymer film Q8 are shown in Fig. 18. Fluorescence derived from the Nile red was not observed in the interior of the film. The test film remained to be colorless transparent even by visual inspection.

### (Experimental Example 8)

A powder of fluorinated copolymer P7 (Solvay Specialty Polymers trade name, Halar 6014) comprising CTFE units and E units, was hot-pressed, to obtain a colorless transparent fluorinated copolymer film Q9 of 67 µm in thickness. The test film for evaluation of drug non-absorbability remained to be colorless transparent by visual observation.

### (Experimental Example 9)

To 100 parts by mass of the fluorinated copolymer P6 synthesized in Experimental Example 7, 1 part by mass of Perkadox 14 (manufactured by Kayaku Akzo Corporation), 3 parts by mass of triallyl isocyanurate, and 1 part by mass of calcium stearate were uniformly kneaded, followed by press-vulcanization at 150°C for 20 minutes, to obtain a fluorinated copolymer film Q10. The type A durometer hardness of the above film was 58, and the Young's modulus was 1.4 MPa.

The hardness was measured in accordance with JIS K6253-3. The Young's modulus was measured three times using a Tensilon (manufactured by A&D Company, Limited) series, by No. 8 dumbbell (width 3 mm, length 10 mm) at a tensile rate of 10 [mm/min], and an average value was adopted.

With reference to the method described in a reference document (Biochemical and Biophysical Research Communications 482 (2017) 323 to 328), using nifedipine and Bay K8644 as drugs, in a 96well plate (manufactured by AGC Techno Glass Co., Ltd.), the polydimethylsiloxane crosslinked product used in Comparative Example 1 punched out so as to have a thickness of 2 mm and a diameter 6.5 mm, was put, and 250 uL of a 1 µM drug solution was added. Up to 3 hours, the drug concentration in the solution was continuously measured, and the absorbed amount was calculated. As a control, the well (TCPS) in which nothing was contained, was used.

The above-mentioned fluorinated copolymer film Q10 was similarly formed into a disk, and in the same manner, the absorbed amount of the drug was calculated. In the case of Nifedipine, by the polydimethylsiloxane crosslinked product, 40% of the drug disappeared from the solution after 3 hours, but by the fluorinated copolymer film Q10, only 10% disappeared. In the case of Bay K8644, by the polydimethylsiloxane crosslinked product, 20% of the drug disappeared after 3 hours, while by the fluorinated copolymer film Q10, no substantial disappearance was observed.

### (Experimental Example 10)

The fluorinated crosslinked film Q1 obtained in Experimental Example 1 was measured in accordance with JIS K7126-1: 2006, whereby the transmission coefficient of oxygen was 1.7×10⁻⁹, and the transmission coefficient of carbon dioxide was 4.0×10⁻⁹. The transmission coefficient of water vapor was measured in accordance with JIS K7129: 2008, whereby the transmission coefficient was 1.0.

In the same manner, the film Q10 made of the fluorinated copolymer obtained in Experimental Example 9 was subjected to measurements of the gas permeability constants, whereby the transmission coefficient of oxygen was 2.0×10⁻¹⁰, and the transmission coefficient of carbon dioxide was 10.0×10⁻¹⁰.

### (Example 1)

Using the fluorinated polymer P1 as described in Experimental Example 1, a 0.6 mm thick film was prepared by hot pressing and subjected to ultraviolet irradiation under the same conditions as in Experimental Example 1 to obtain a fluorinated crosslinked film Q10. From this, three sheets of rectangular film of 20 mm × 40 mm were cut out by a blade. In the first sheet, two through holes with a diameter of 2 mm were formed by a punch from the irradiated surface side (hole center distance was 20 mm). In the second sheet, two through holes were formed in the same operation at the same positions as in the first sheet, and further, by using a blade, a through-groove with a line width of 1 mm, connecting between these through holes, was formed. The two sheets were laminated so as to match the positions of the through holes in the first sheet and the second sheet. Further, the third sheet was placed below the second sheet, so that the irradiated surface became the opposite side to the second sheet, followed by lamination so that the four vertices of the rectangles would overlap. Under reduced pressure under vacuum, the three sheets were heated and bonded at 100°C for 3 hours, to prepare a chip structure having an inner space that connects the two openings.

### (Example 2) [Cell culture test]

The above fluorinated copolymer film Q10 was subjected to molding, to prepare a film with a thickness of 1.5 mm having a bottomed groove with a height 0.2 mm and a width of 1.0 mm. The film having predetermined positions punched out by a 2 mmϕ Biopsy Punch (manufactured by Kai Corporation) adopted as a first substrate, and the film having no through hole drilled, adopted as a second substrate, were laminated so that the center grooves would face each other.

Between said first and second substrates, as shown in Fig. 10, a PDMS (polydimethylsiloxane) film with a thickness of 10 µm was sandwiched so as to separate the center grooves, and a PDMS glue was applied, followed by lamination to prepare a chip. The chip was sterilized with UV for 30 minutes, and a fibronectin solution was injected from a liquid feed port via a syringe to coat the PDMS film. By injecting PBS from the liquid feed port via a syringe, the PDMS film was washed. From the liquid feed port via a syringe, GFP expression HUVEC (vascular endothelial cells) suspended in a culture medium, was injected and incubated at a temperature of 37°C in a 5% CO₂ environment for 16 hours.

The cells (HUVEC) after the culturing for 16 hours were observed by microscopic observation, whereby the cells were clearly observable in the phase difference image and the GFP fluorescence image. Further, the cell storage portion was subjected to expansion and contraction to give a stretch stimulus to the cells, whereby it was possible to present a stretch stimulus of about 5% to the cells.

Therefore, it has been found that the chip was provided with the drug non-absorbability, cell nontoxicity, cell observability and flexibility, required for a biomimetic chip whereby the evaluation can be conducted in an environment close to the living body, and thus, it can be suitably used as a biomimetic chip.

### (Example 3)

The above-mentioned polydimethyl siloxane crosslinked film Q2 was molded by using a mold made of a silicon substrate and a resist resin, to prepare a film of 6 mm in thickness having a bottomed groove with a height of 0.2 mm and a width of 1.0 mm. To this film, fluorinated copolymer P1 was coated in the same manner as in Experimental Example 2 to obtain a fluorinated copolymer coating film Q13. However, UV irradiation was carried out under the atmospheric air. In Example 2, in place of the film Q10 having a bottomed groove, the film Q13 having a bottomed groove was used, and in place of the PDMS film having a thickness of 10 µm, a Collagen Vitrigel (AGC Techno Glass Co., Ltd. trade name) film, was used, and they were laminated without applying a PDMS glue, and pressed at 30°C, to prepare a chip. It is possible to grow cells on Vitrigel, and thus, it has been found that the chip can be suitably used as a biomimetic chip.

### (Example 4)

The fluorinated crosslinked film Q1 was obtained in the same manner as in Experimental Example 1, except that UV irradiation was conducted in the atmospheric air. This was placed in a polystyrene petri dish, and TIG-3 cells being human fetal lung-derived fibroblast cells, were cultured on the film Q1 for 3 days. Cell adhesion was good.

From the foregoing results, it has been found that the films Q1, Q5 to Q9 made of the fluorinated copolymers P1, P3 to P7 are excellent in drug non-absorbability, and thus can be suitably used as substrates of chips to be used in the evaluation tests of drugs.

The polydimethylsiloxane crosslinked film Q2 absorbs drugs, but the fluorinated copolymer coated film Q3 having a coating layer of the fluorinated copolymer P1 provided on this film, and the fluorinated copolymer coated film Q4 having a coating layer of the fluorinated copolymer P2 provided thereon, are excellent in drug non-absorbability, and thus can be suitably used as substrates of chips to be used in the evaluation tests of drugs.

The entire disclosure of Japanese Patent Application No. 2017-177979 filed on September 15, 2017 including specification, claims, drawings and summary is incorporated herein by reference in its entirety.

### REFERENCE SYMBOLS

1: first substrate, 2: second substrate, 3: third substrate, H1, H2, H11, H12: openings, S: inner space, S1, S2, S11, S12: through holes, S3: through groove, S13: bottomed groove (bottom-equipped groove), 1a, 1b, 11a, 11b: inner wall surfaces of through holes, 1c: surface facing the through groove, 2a: inner wall surface of the through groove, 3a: surface facing the through groove, 11c: surface facing the bottomed groove, 12a: inner wall surface of the bottomed groove, 12b: bottom surface of the bottomed groove, 21: first substrate, 22: second substrate, S21, S22: through holes, S23: through groove, 21a, 21b: inner wall surfaces of the through holes, 21c: surface facing the through groove, H21, H22: openings, 31: first substrate, 32: second substrate, 33: film, S31, S32, S33, S34: through holes, S35: bottomed groove, 31a, 31b, 31c, 31d: inner wall surfaces of the through holes, 32a, 32b: inner wall surfaces of the bottomed grooves, H31, H32, H33, H34: openings

## Claims

1. A microchannel chip having formed a plurality of openings and an inner space connecting the plurality of openings, wherein at least a part of the surface in contact with the inner space is made of a fluorinated copolymer containing from 5 to 99 mol% of units based on CF₂=CFX (X is a fluorine atom, a chlorine atom or CF₃), to all units in the copolymer.

2. The microchannel chip according to Claim 1, wherein the inner space includes a storage portion for storing cells.

3. The microchannel chip according to Claim 1 or 2, wherein at least a first substrate, a second substrate and a third substrate are laminated in this order, wherein in the first substrate, a plurality of through holes are formed, and in the second substrate, a through groove penetrating from the front surface to the back surface is formed so as to connect the plurality of through holes of the first substrate,
at least one of the first substrate, the second substrate and the third substrate is made of the fluorinated copolymer, or
at least a part of the inner wall surfaces of the plurality of through holes and the surface facing the through groove, of the first substrate, the inner wall surface of the through groove of the second substrate, and the surface facing the through groove, of the third substrate, is coated with the fluorinated copolymer.

4. The microchannel chip according to Claim 3, wherein the inner space formed by the plurality of through holes and the through groove, incudes a storage portion for storing cells.

5. The microchannel chip according to Claim 1 or 2, wherein at least a first substrate and a second substrate are laminated, wherein in the first substrate, a plurality of through holes are formed, and in the second substrate, a bottomed groove is formed so as to connect the plurality of through holes of the first substrate,
at least one of the first substrate and the second substrate is made of the fluorinated copolymer, or
at least a part of the inner wall surfaces of the plurality of through holes, and the surface facing the groove, of the first substrate, and the bottom surface and inner wall surface of the groove of the second substrate, is coated with the fluorinated copolymer.

6. The microchannel chip according to Claim 5, wherein the inner space formed by the plurality of through holes and the bottomed groove, incudes a storage portion for storing cells.

7. The microchannel chip according to Claim 1 or 2, wherein at least a first substrate and a second substrate are laminated, wherein in the first substrate, a plurality of through holes are formed and a bottomed groove is disposed so as to connect the through holes, and in the second substrate, no hole or groove is formed,
at least a part of the inner wall surfaces of the plurality of through holes and the surface facing the groove, of the first substrate, is made of the fluorinated copolymer, or
at least a part of the inner wall surfaces of the plurality of through holes and the surface facing the groove, of the first substrate, is coated with the fluorinated copolymer.

8. The microchannel chip according to Claim 1 or 2, wherein at least a first substrate and a second substrate are laminated, wherein in the first substrate, a plurality of through holes and a bottomed groove are formed, and in the second substrate, a bottomed groove is disposed so as to connect the plurality of through holes of the first substrate, and between the first and second substrate, a film is disposed so as not to block the through holes,
at least a part of the inner wall surfaces of the plurality of through holes and the surface facing the bottomed groove, of the first substrate, and the bottom surface and inner wall surface of the groove of the second substrate, is made of the fluorinated copolymer, or
at least a part of the inner wall surfaces of the plurality of through holes of the first substrate, and the bottom surface and inner wall surface of the groove of the second substrate, is coated with the fluorinated copolymer.

9. The microchannel chip according to Claim 8, wherein the film as defined in Claim 8 is made of collagen, polydimethylsiloxane, or a fluorinated copolymer containing from 5 to 99 mol% of units based on CF₂=CFX (X is a fluorine atom, a chlorine atom or CF₃).

10. The microchannel chip according to any one of Claims 1 to 9, wherein the fluorinated copolymer is a copolymer having from 50 to 80 mol% of units based on tetrafluoroethylene, the transmission coefficient of oxygen is from 1.0 to 390×10⁻¹⁰ [cm^{3∗} cm/(cm^{2∗}s^{∗}cmHg)], and the transmission coefficient of carbon dioxide is from 5.0 to 1,900×10⁻¹⁰[cm^{3∗}cm/(cm^{2∗}s^{∗}cmHg)].

11. The microchannel chip according to any one of Claims 1 to 10, wherein the fluorinated copolymer is a copolymer containing from 50 to 70 mol% of units based on tetrafluoroethylene, the type A durometer hardness is at least 80, the Young's modulus is at most 1.5 MPa, the nifedipine absorptivity is at most 30%, the BayK8644 absorptivity is at most 10%, and the inner space of the chip can be visually confirmed.

12. The microchannel chip according to any one of Claims 1 to 11, wherein the units based on said CF₂=CFX are units based on tetrafluoroethylene, units based on hexafluoropropylene, or units based on chlorotrifluoroethylene.

13. The microchannel chip according to any one of Claims 1 to 12, wherein the fluorinated copolymer further contains at least one type selected from the group consisting of units based on propylene, units based on ethylene, units based on vinylidene fluoride, and units based on a perfluoro(alkyl vinyl ether), in an amount of from 1 to 95 mol% to all units which the polymer comprises.

14. The microchannel chip according to any one of Claims 1 to 13, wherein the fluorinated copolymer is either
a copolymer comprising units based on tetrafluoroethylene and units based on propylene, and their total is from 65 to 100 mol% to all units,
a copolymer comprising units based on tetrafluoroethylene and units based on ethylene, and their total is from 80 to 100 mol% to all units,
a copolymer comprising units based on hexafluoropropylene and units based on vinylidene fluoride, and their total is from 50 to 100 mol% to all units,
a copolymer comprising units based on chlorotrifluoroethylene and units based on ethylene, and their total is from 80 to 100 mol% to all units,
a copolymer comprising units based on tetrafluoroethylene and units based on a perfluoro(alkyl vinyl ether), and their total is from 50 to 100 mol%, or
a copolymer comprising units based on tetrafluoroethylene and units based on hexafluoropropylene, and their total is from 50 to 100 mol% to all units.

15. The microchannel chip according to any one of Claims 1 to 14, wherein the fluorinated copolymer has from 0.01 to 5.0 mass% of iodine atoms, to the copolymer.

16. The microchannel chip according to any one of Claims 1 to 15, wherein the fluorinated copolymer
comprises units based on tetrafluoroethylene and units based on propylene, and their total is from 65 to 100 mol% to all units,
the molar ratio of units based on tetrafluoroethylene/units based on propylene is from 30/70 to 70/30, and the copolymer contains from 0.01 to 5.0 mass% of iodine atoms.

17. The microchannel chip according to any one of Claims 1 to 16, wherein the fluorinated copolymer
comprises units based on tetrafluoroethylene and units based on a perfluoro(alkyl vinyl ether), and their total is from 50 to 100 mol% to all units,
the molar ratio of units based on tetrafluoroethylene/units based on a perfluoro(alkyl vinyl ether) is from 5/95 to 95/5, and
the copolymer contains from 0.1 to 20 mol% of units derived from a monomer III represented by the following formula (III),
CR¹¹R¹²=CF-Q-R¹³-CO-Z (III)
(in the formula (III), R¹¹ and R¹² are each independently a hydrogen atom or a fluorine atom, Q is a single a bond or an etheric oxygen atom, R¹³ is a group having an etheric oxygen atom at least at one terminal or between a carbon-carbon bond of a fluoroalkylene group or two or more fluoroalkylene groups, -Z is -OH, -OR¹⁴, -NR¹⁵R¹⁶, -NR¹⁷NR¹⁸H or NR¹⁹OR²⁰, R¹⁴ is an alkyl group, and R¹⁵, R¹⁶, R¹⁷, R¹⁸, R¹⁹ and R²⁰ are each independently a hydrogen atom or an alkyl group.)

18. The microchannel chip according to any one of Claims 1 to 17, which contains cells in the inner space.

19. The microchannel chip according to Claim 18, wherein the fluorinated copolymer is a copolymer containing from 50 to 70 mol% of units based on tetrafluoroethylene, and elongation of the cell culture surface in the state of containing cells in the inner space is from 1.0 to 25.0%.
